# EUROPEAN PATENT APPLICATION

(11) **EP 3 216 378 A1**
(43) Date of publication of application: **13.09.2017**
(21) Application number: 16159067.4
(22) Date of filing: 08.03.2016
(51) Int. Cl.: A47L 15/42, A61L 2/00, C07F 11/00, D06F 35/00

(54) **HOUSEHOLD APPLIANCE WITH A POLYOXOMETALATE-CONTAINING SURFACE AND METHOD FOR OPERATING THE SAME**

(71) Applicant: BSH Hausgeräte GmbH, 81739 München (DE)
(72) Inventor: Barrado Franco, Antonio, 10317 Berlin (DE); Bischof, Andreas, 10407 Berlin (DE); Hanau, Andreas, 12359 Berlin (DE); Schaub, Hartmut, 14656 Brieselang (DE)

(57) **Abstract**

The invention relates to a household appliance 1 with at least one polyoxometalate containing surface 17, wherein the catalytically active substance is a polyoxometalate of the Formula (I) or (II),

A_{q+3}PV_{q}Z_{12-q}O₄₀ (I),

A₃PZ₄O₂₄ (II)

wherein Z is selected from Mo oder W,
q is 0, 1 , 2 or 3, and
A is selected from one or more cations selected from the group consisting of (XR¹R²R³R⁴)⁺, wherein at least one of the groups R¹, R², R³, and R⁴ is a substituted or nonsubstituted alkyl or cycloalkyl group with at least five carbon atoms and wherein X is selected from nitrogen or phosphorus. The invention relates moreover to a method for operating a household appliance of this kind.

## Description

The invention relates to a household appliance with a polyoxometalate-containing surface and a method for operating the same. In particular, the invention relates to a household appliance with at least one surface comprising a specific polyoxometalate and a method for operating the same.

Household appliances can come into contact with dust, dirt, food, humidity or human skin. This may lead to hygienic problems in the long term, since microorganisms may deposit on the household appliance and may proliferate.

Moreover, articles which have been soiled in various ways are in general cleaned in water-bearing household appliances. Food remains occur in dishwashers and the range of dirt in laundry items to be cleaned in washing machines is typically even greater. All water-bearing household appliances have in common that in the humid and warm atmosphere, in particular at sites that are difficult to access, dirt can arise and accumulate. This dirt may be a good nutrient medium for microorganisms such as bacteria and fungi.

A hygiene problem may exist particularly where water remains in a household appliance. Therefore, in a laundry treatment device, for example, a washing machine, when a water reservoir is used or on frequent use of washing programs with cold suds, during long phases of non-use with the door closed or under unfavorable placing conditions, dirt can accumulate, particularly the formation of biofilms comprising organic substances such as microorganisms and nutrients. Such biofilms lead to bad odors and/or visible dirt. Pathological effects may even occur. Biofilms form particularly in areas of washing machines in which water is present for long periods, i.e. during phases of non-use. Such areas include poorly ventilated areas such as ribs, pockets, folds in seals, water inflows and outflows and, most particularly, water reservoirs which serve to store the greywater arising in the washing and rinsing process for re-use in a later washing or rinsing process. It is therefore desirable to sanitize the water in a household appliance and to prevent the formation of biofilms.

Other household appliances that may tend to contaminations with microorganisms are for example kitchen hoods or vacuum cleaners.

Irrespective of the type of the household appliance, a problem arises in that for controlling the household appliance an operating panel must be touched in general by a user. In this way operating panels can be contaminated with microorganisms which may moreover be spread when the household appliance is used by several people.

In order to maintain a hygienically impeccable state in a household appliance various methods are currently being used. In particular, various measures for removing and/or preventing biofilms are known from the prior art. In water-bearing household appliances, sometimes machine cleaning programs are offered which remove built-up of dirt at high temperatures with the assistance of washing agents and sometimes with increased suds levels and/or at raised drum rotations speeds, i.e. with an increased input of mechanical energy. Also known is the use of ozone to remove organic dirt.

Among the best known methods, the use of antimicrobial active ingredients in the surface, for example silver ions, is envisaged. These have however the disadvantage that they are gradually removed from the surface and are thus depleted. Moreover, silver is one of the heavy metals which are detrimental to the environment. In the case of killing microorganisms in water-bearing household appliances using Ag+ or Cu+ ions in the washing suds and on the surfaces of the materials coming into contact with the washing suds, disadvantageous effects with regard to groundwater and waterway pollution also arise. However, if these active ingredients are not washed out from a carrier material in the surfaces of the household appliance, their efficacy is mostly small such that over a long term microorganisms and even biofilms are being formed on the surface and negatively affect the hygienic state of the household appliance.

It has also previously been proposed to remove microorganisms with the aid of UV-C-radiators using a thorough-flow principle, the removal taking place in such a manner that microorganisms in organic dirt are finally killed by damaging their genetic material.

Other methods are directed to thermal killing of any microorganisms present by increasing the temperature at the surfaces of the components coming into contact with the washing suds, via direct or indirect energy transfer (water, steam, microwaves).

Photocatalytic methods are also known, for example, the use of catalytically effective compounds, in particular titanium dioxide coatings for deodorizing, disinfecting and cleaning. In this process, the catalyst needs to be often activated by means of UV radiation. These compounds support the oxidative modification or destruction of microorganisms, such that they are removed in the best case in total by oxidation. Often this method works however only in a limited manner, in particular if UV light is required in addition for the oxidation.

A disadvantage of these known methods and measures is the high energy usage and the sometimes high apparatus and/or operating costs for achieving remarkable effects. In some methods, potentially health-endangering agents, for example ozone or UV radiation, are used so that additional safety measures are required.

Specific surface structures which make use of the lotus effect aim at reducing the adhesion of dirt and at easing the cleaning of the surfaces. There is in general no antimicrobial effect of these surfaces. A big disadvantage of all these methods is that they all operate very locally. Thus, there is no long range antimicrobial effect in the free water of a water storage container.

The use of polyoxometalates in various fields is known, for example, in analytical and clinical chemistry, in catalysis (including photocatalysis), in biochemistry (inhibition of electron transfer processes), in medicine (antitumor and antiviral activity) and in the manufacturing of integrated circuits. Polyoxometalates are known as oxidation catalysts most of all in the paper and plastic industry.

The publication EP 1 141 210 B1 discloses a method for bleaching laundry items or household surfaces wherein a washing agent which contains polyoxometalates is brought into contact with the soiled substrate. Air serves as the primary source of oxygen atoms for bleaching.

The publication DE 10 2009 026 712 A1 discloses a household appliance having at least one component which has a surface that can be affected by organic dirt, said surface having a photocatalyst, a photosource for irradiating the photocatalyst with an activating electromagnetic radiation being associated with said surface, the surface being formed from a primary formed first materials in which the photocatalyst is dispersed. Materials with titanium dioxide and modifications thereof are disclosed in great detail as photocatalysts.

The publication EP 2 761 073 B1 discloses a water-bearing domestic appliance having a container for receiving objects to be cleaned and at least one inner surface containing a catalytically active substance, said surface being disposed inside the domestic appliance, wherein the catalytically active substance is a polyoxometalate and the inner surface comes into contact with water to be cleaned during operation of the domestic appliance. Preferably the polyoxometalate is a tungstate and even more preferably the tungstate is modified by titanium.

The publication WO 2014/154432 A1 discloses a household appliance which comprises at least one catalytically effective substance in a surface, wherein the catalytically effective substance is a polyoxometalate that is comprised in an inner and/or outer surface of the household appliance, provided that the polyoxometalate is comprised at least in an outer surface of the household appliance if the household appliance is a water-bearing household appliance having a container for receiving objects to be cleaned.

The publication WO 2014/122225 A1 discloses the use of a heteropolyoxometalate of the Formula (I), (II) or (III)

A_{q+3}PV_{q}Z_{12-q}O₄₀ (I),

A₆P₂Z₁₈O₆₂ (II),

or

A₃PZ₄O₂₄ (III)

wherein Z is selected from Mo or W,
index q = 0, 1, 2 or 3, and

A is selected from one or more cations and comprises at least one cation selected from the group consisting of quaternary ammonium cations, quaternary phosphonium cations and tertiary sulfonium cations for providing self-cleaning, stripping, disinfecting, self-sanitizing, biocidal, antimicrobial, and/or deodorizing properties to at least part of a substrate or a surface of a substrate or to a coating or for decomposition and/or degradation of organic materials. In Formula (I), Z is preferably Mo and q = 2, and in Formula (III), Z is preferably W.

The publication EP 2765 136 A1 discloses a heteropolyoxometalate of the Formula (I), (II) or (III)

A_{q+3}PV_{q}Z_{12-q}O₄₀ (I),

A₆P₂Z₁₈O₆₂ (II),

or

A₃PZ₄O₂₄ (III)

wherein Z is selected from Mo or W,
q is 1, 2 or 3, and

A is selected among one or more cations and comprises at least one quaternary ammonium cation with the proviso that the compounds [(n-C₄H₉)₄N]₃PMo₁₂O₄₀ and [(n-C₆H₉)₄N]₃PMo₁₂O₄₀ are exempted. Most preferred are the heteropolyoxometalates [(n-C₄H₉)₄N]₃PW₄O₂₄ and [(n-C₆H₁₃)₄N]₃PW₄O₂₄.

It is an object of the present invention against this background, to provide a household appliance and a method for operating said appliance wherein the growth of microorganisms on or in the household appliance is prevented or at least hindered. Preferably, dirt can be removed or prevented in a simple and particularly hygienic way or at least largely removed or prevented. It should be preferably possible to remove or prevent dirt with microorganisms. The maintenance of a hygienic state should thus be easier possible since the cleaning effort should be less with regard to the extent and the required number of repetitions of cleaning steps. Ideally, cleaning should be avoidable in total.

This object is achieved according to the invention with a household appliance and a method for operating the same, having the features of the corresponding independent claims. Preferred embodiments of the household appliance according to the invention are disclosed in the corresponding dependent claims. Preferred embodiments of the household appliance according to the invention correspond to preferred embodiments of the method according to the invention and vice versa, even if not explicitly stated herein.

The invention is therefore directed to a household appliance, preferably a water-bearing household appliance comprising a tub for accommodating articles to be cleaned, with at least one polyoxometalate containing surface, wherein the catalytically active substance is a polyoxometalate of the Formula (I) or (II),

A_{q+3}PV_{q}Z_{12-q}O₄₀ (I),

A₃PZ₄O₂₄ (II)

wherein Z is selected from Mo or W, q is 0, 1, 2 or 3, and

A is selected from one or more cations selected from the group consisting of (XR¹R²R³R⁴)⁺, wherein at least one of the groups R¹, R², R³, and R⁴ is a substituted or nonsubstituted alkyl or cycloalkyl group with at least five, preferably at least six and even more preferably at least seven, carbon atoms and wherein X is selected from nitrogen or phosphorus.

Preferably, the polyoxometalate is a polyoxometalate of the Formula (I'), (II') or (III'),

A_{q+3}PV_{q}Mo_{12-q}O₄₀ (I'),

A₃PW₄O₂₄ (II'),

or

A_{q+3}PV_{q}W_{12-q}O₄₀ (III'),

wherein q is 0, 1 or 2.

More preferably, the polyoxometalate is

A_{q+3}PV_{q}Mo_{12-q}O₄₀ (I'),

or

A_{q+3}PV_{q}W_{12-q}O₄₀ (III'),

wherein q is 1 or 2.

In a particular preferred embodiment of the household appliance, the polyoxometalate is

A₃PW₄O₂₄ (II').

The household appliance is not limited. However, the household appliance is preferably a water-bearing household appliance. In general, a water-bearing household appliance is a household appliance during the operation of which water is used. The items to be cleaned can be, in particular, tableware or laundry items. Cleaning should according to the invention also be understood to mean freshening.

In the case of a water-bearing household appliance it is preferable that the surface is an interior surface provided in the interior of the household appliance which comes into contact with flowing or static water during operation of the household appliance. The contact is usually necessary, since organic substances in the water of the household appliance are oxidized due to the contact with the polyoxometalate-containing surface and are thereby decomposed.

In the case of a water-bearing household appliance it is preferable that at least one interior surface is provided in a water feed system. In addition or alternatively it is preferable that the at least one interior surface is provided in a water reservoir. It is then advantageous that the water reservoir comprises a circulating element and/or an air infeed element.

In a preferred embodiment, the water-bearing household appliance is a dishwasher or a laundry treatment device or a coffee making machine.

In another preferred embodiment, the water-bearing household appliance is a laundry treatment device belonging to the group consisting of a washing machine and a washer-dryer.

The inventive household appliance enables for example the water in a water-bearing household appliance to be treated in an efficient, economical and environmentally-friendly way such that organic substances like microorganisms and nutrients are decomposed and therefore a biofilm in the household appliance can be counteracted. In this way, the property of the polyoxometalates, and in particular of the specific polyoxometalates as described herein, as oxidation catalyst or as oxidation agent is utilized. Namely, the effects of the polyoxometalate can be two-fold, the extent of these effects being dependent on the specific polyoxometalate used.

For example, a cause of disinfection might be an oxidation of organic substances by the polyoxometalate itself when they are in direct contact. Then the polyoxometalate would be itself reduced. Such an oxidation may lead depending on the extent of oxidation to the partial or complete transformation of the microorganisms. Sometimes even a minor oxidative impact on the microorganism might be sufficient, if the microorganism can be transformed into a state wherein reproduction is no longer possible.

More importantly, however, the polyoxometalate containing surface is catalytically effective in oxidation reactions. In the oxidation reaction, the polyoxometalate serves, in general, as an oxidation catalyst which cooperates with an oxidizing agent. The oxidizing agent is not limited according to the invention. Preferably, an oxygen-containing oxidizing agent is used. Oxygen, inorganic or organic peroxides and/or ozone are particularly preferred as oxidizing agents. Oxygen is again particularly preferred amongst them as an oxidizing agent since in this manner, an additional input of possibly harmful or interfering substances can be avoided. Air is preferably used as a source of oxygen. However, the oxygen may be present, for example, dissolved in water. Furthermore, by means of a corresponding movement of the water, mixing of air and water can take place, for example, for the formation of air bubbles in the water and an air-water mixture of this type can be brought into contact with the polyoxometalate-containing surface, the atmospheric oxygen preferably serving as the oxidizing agent. Particularly preferably, an air infeed element provides a sufficient quantity of oxygen in the water to be treated.

In a further preferred embodiment, a peroxide or ozone is used as the oxidizing agent.

Ozone is preferred as an oxidizing agent in a household appliance having an ozone generator. Such a household appliance preferably has also an ozone removal device. The polyoxometalate-containing surface is then arranged such that said surface can come into contact with water and ozone. For example, in a washing machine in which ozone can be introduced into the outer tub, the polyoxometalate-containing surface is arranged in the outer tub accordingly.

If a peroxide is used as the oxidizing agent, preferably, peroxide already present in the laundry care product is used. This saves the addition of further peroxide.

In a preferred household appliance of the present invention, in particular a water-bearing household appliance, and more particularly a washing machine or a wash-dryer, the polyoxometalate-containing surface is arranged in the detergent dispenser tray or in the outer tub, particularly preferably in the outer tub if peroxides serve as the oxidizing agent. The reason therefore is that peroxides are present particularly in heavy-duty washing agents and bleaching agents, although bleaching agents in particular are often added directly in the drum.

Preferably, the user of the laundry treatment device according to an embodiment of the household appliance of the present invention can specify, by means of selection buttons, whether heavy-duty washing agents or bleaching agents have been added. Alternatively, the presence of strong oxidizing agents such as peroxides or ozone can be established by means of one or more sensors in the washing machine. In the presence of ozone or peroxides, the operation of a circulating element or an air infeed element can be dispensed with.

Thus, in the presence of an oxidizing agent such as oxygen, hydrogen peroxide or ozone, oxygen radicals (or oxygen-containing radicals, herein referred altogether as "oxygen radicals") are formed at the polyoxometalate-containing surface of the household appliance, whether it is an internal or external surface. When these highly reactive oxygen radicals come into contact with organic substances including microorganisms, an oxidation reaction takes place which leads to the decomposition of the organic substances and/or to pathological processes in the microorganisms. In this way, the formation of a biofilm is counteracted. For example organic substances in the water which is brought into contact with the interior surface of a water-bearing household appliance coated with the polyoxometalate are decomposed and therefore the formation of a biofilm is hindered.

In order to enable a continuous oxidation reaction, a sufficient level of mobility of the oxidizing species, for example, of oxygen radicals which have been generated at the polyoxometalate-containing surface acting as an oxidation catalyst should be provided. This could be for example achieved by recirculating an aqueous medium with the aid of a pump. In cases where there is in general no flow around the polyoxometalate-containing surface, it might be sufficient to exploit natural diffusion, or convection in case of temperature differences. It might be possible to dispense with a sufficient mobility at the surface when the desired disinfection is to be achieved first of all at a surface itself, for example hygienic control elements, and where a long range effect, for example a complete disinfection of the surrounding medium, by means of reactive oxygen species can be dispensed with.

Furthermore, a sufficiently high concentration of oxidizing agent should be present in the water to be treated in order to achieve a sufficient hygienic effect.

In the household appliance of the present invention, the cation is preferably selected from one or more cations selected from the group consisting of (NR¹R²R³R⁴)⁺, wherein at least one of the groups R¹, R², R³, and R⁴ is a substituted or nonsubstituted alkyl or cycloalkyl group with at least eight carbon atoms.

In another preferred embodiment, the cation is selected from one or more cations selected from the group consisting of (PR¹R²R³R⁴)⁺, wherein at least one of the groups R¹, R², R³, and R⁴ is a substituted or nonsubstituted alkyl or cycloalkyl group with at least eight carbon atoms.

In the household appliance of the present invention it is preferred that the surface has a polyoxometalate containing upper layer with a thickness of from 0.01 to 1.5 mm, preferably a thickness of from 0.02 to 1 mm, and even more preferably from 0.05 to 0.5 mm.

It is moreover preferred that in the household appliance the upper layer of the surface has a polyoxometalate content in the range of from 0.5 to 50 wt%, more preferably in the range of from 1 to 15 wt%, and even more preferably in the range of from 1 to 10 wt%, based on the weight of the upper layer (in the following also to be termed "surface layer").

Namely, it is advantageous if as much as possible polyoxometalate is present in the polyoxometalate containing surface, since its effect is in general restricted to the surface. This is especially the case when the purpose of the application of the polyoxometalate is not only a disinfected surface, but also a disinfection of the surrounding medium. For sur faces where the aim is especially a disinfected surface, less polyoxometalate may be used, for example a 10 wt% content in the surface layer, perhaps even 1 wt% might be sufficient, based on the weight of the surface layer.

The polyoxometalate-containing surface can be created in any way, provided the catalytic effect according to the invention is possible. Thus, for example, said surface can be created by forming a polyoxometalate-containing film or, for example, by placing polyoxometalate particles at the surface of a porous material. The production thereof depends, in particular on the location of the surface and the manner of using the household appliance.

In order to maintain the polyoxometalate at the surface and to prevent a dissolution into a surrounding liquid medium or any other depletion because of the contact with a surrounding medium, it is important to use the specific cations described herein. Organic ions with a long chain are especially suitable as described herein. Particularly preferred examples thereof are the methyltrioctylammonium and tributyltetradecylphosphonium cations.

The polyoxometalate described herein is a salt which can be bound by means of suitable binder systems on various surfaces. Alternatively, the polyoxometalate may be admixed with the remainder of the material in the surface, ahead of the manufacture of the surface bearing item, for example an organic polymeric material. But it is also possible to use a mixture of the heteropolyoxometalate with a polymeric material and use the mixture to for entire building parts.

Moreover, it is possible to produce a foil wherein the polyoxometalate is combined with a suitable carrier material, such that only a comparatively small amount of polyoxometalate might be required in order to cover a large surface area. Such foils can be applied to relevant parts in the household appliance, for example by gluing or melt bonding. Moreover such foils may be placed directly in an injection mould and thus directly utilized for the manufacture of parts of the household appliance.

However, in the household appliance according to the invention, any surface can contain polyoxometalates. These surfaces can be interior or exterior surfaces.

The polyoxometalate-containing surfaces may be outer surfaces of handles, knobs, handle bars, controls, or of switches, for example light switches, handrails, sliders, control dials, slide controls or touch sensitive (sensor active) surfaces.

In a dryer as a household appliance according to the present invention, the surface can be for example the container accepting items to be dried and the condensate container. In general all water-bearing parts in a household appliance, for example pumps including their housing, tubing, valves can contain polyoxometalate-containing surfaces.

An interior polyoxometalate containing surface might be an inner wall of a water container and/or of a flow-through part of a water-bearing household appliance.

The water container can be for example the water container of a coffee percolator. Moreover, all other parts of the percolator coming into contact with water or milk etc. can have polyoxometalate-containing surfaces.

Household appliances according to the present invention are moreover containers wherein waste, garbage or used, spoiled food is temporarily stored.

Other examples where the invention can be utilized are for the disinfection of the surfaces in scales or balances, in order to avoid the transfer of pathogenic microorganisms.

In the case where the household appliance is a washing machine, it is especially advantageous if the outer tub has surfaces that contain a polyoxometalate. The formation of biofilms is avoided and moreover leaching of a biofilm may be activated in the course of a washing program. Another part in a washing machine is the detergent dispenser tray wherein also biofilm formation can be avoided.

In a dishwasher as household appliance of the present invention a polyoxometalate containing surface may assist in the activation of bleaches, in the decomposition of organic material and the disinfection of both running and stored water.

In cooling devices which comprise herein broadly also freezers, the invention can be used for the disinfection of inner surfaces and of condensate which might be present on the inner surfaces as well as in the condensate container and tubing.

The household appliance according to the invention can also be a vacuum cleaner or a device for the cleaning or maintenance of a floor, wherein the polyoxometalate containing surfaces are especially in the area of filters and nonwovens and the anther as well as the device for holding the anther.

The inventive household appliance can be moreover an oven, a microwave device or a steamer, a stove or a stovetop, whereby the surface can be hygienized because of a content in the polyoxometalate described herein.

Further examples for household appliances in accordance with the present invention are flow-type heaters; vapour stations and steam irons; air humidifiers and air conditioning devices. In these cases, the disinfection of the surfaces as well as of the water to be heated is of importance. In general it is also here important that by means of the disinfection of the surfaces, the transmission of possibly harmful microorganisms or fungi can be avoided in case of skin contact.

In the case of kitchen hoods as a further example for a household appliance according to the present invention the disinfection of the surfaces, in particular of the meshes and of the filter mats, as well as the disinfecting treatment of the air is of importance.

Another important use is in surfaces of filter units.

The invention is moreover directed to a method for operating a household appliance, wherein the household appliance is a water-bearing household appliance comprising a tub for accommodating articles to be cleaned, for example laundry items, and at least one catalytically active polyoxometalate-containing interior surface, which can come into contact with water or washing suds during operation of the household appliance, and wherein the polyoxometalate is of the Formula (I) or (II),

A_{q+3}PV_{q}Z_{12-q}O₄₀ (I),

A₃PZ₄O₂₄ (II)

wherein Z is selected from Mo or W, q is 0, 1, 2 or 3, and A is selected from one or more cations selected from the group consisting of (XR¹R²R³R⁴)⁺, wherein at least one of the groups R¹, R², R³, and R⁴ is a substituted or nonsubstituted alkyl or cycloalkyl group with at least five carbon atoms and wherein X is selected from nitrogen or phosphorus, and wherein water or washing suds to be cleaned is brought into contact with the polyoxometalate-containing surface.

The invention has the advantage that a household appliance, in particular a water-bearing household appliance which has a markedly lower susceptibility to dirt, particularly involving microorganisms is made available in a simple and cost-effective manner and is easy to clean should dirt nevertheless arise.

A particular advantage of the use of the polyoxometalates described herein in surfaces, in particular interior surfaces, lies therein that said surfaces are able to function for years as a catalytic system without any additional activation or addition energy usage being necessary. Only on the use of an additional circulating element or air infeed element would additional energy consumption occur, although compared with other water-processing method such as membrane filtration or electrochemical processes, this would be slight. Furthermore, no addition reagents need to be dosed in and activation of the catalysts, for example, by UV radiation can be dispensed with. Additional advantages of polyoxometalate-containing surfaces lie in the improved effectiveness of bleaching agents through the activation thereof, if used. Furthermore, the polyoxometalates described herein can have virucidal effects. Therefore, with the present invention, a water-processing system for household appliances having almost no ongoing costs and a long service life is provided.

Cleaning is less elaborate due to the specific surfaces. In particular it is possible to clean with a lower frequency or even not at all. The invention provides moreover a long range effect in the sense that not only polyoxometalate containing surfaces are protected or may be freed from microorganisms. The oxidation reactions taking place at polyoxometalate containing surfaces can in addition affect also neighboring areas in a household appliance where they can fight microorganisms.

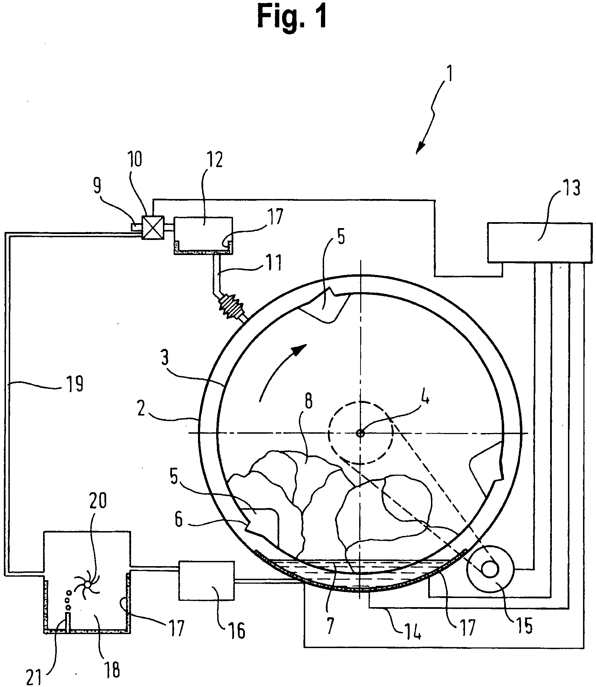

In the following a preferred embodiment of the invention will be described in more detail by reference to Fig. 1 of the drawing atached.

Fig. 1 is a schematic representation of the present relevant parts of an embodiment of the household appliance configured as a washing machine, as non-limiting example.

The washing machine 1 of this embodiment has an outer tub 2 in which a drum 3 is rotatably mounted and can be driven by a drive motor 15. For ergonomic reasons, the rotation axis 4 of the drum 3 is oriented upwardly out of the horizontal by a small angle, so that easier access to, and inspection of the interior of the drum 3 is provided. With this arrangement, in cooperation with specially formed laundry agitators 5 and scooping devices 6 for the washing suds 7 at the internal surface of the drum jacket, intensification of the flow of washing suds 7 through the laundry items 8 can be achieved.

The washing machine 1 also has a water feed system which comprises a water connection fixture for the domestic water supply 9, an electrically controllable valve 10 and a feed pipe 11 which extend to the outer tub 2 and is fed via a detergent dispenser tray 12 from which the feed liquid is able to transport washing agent portions to the outer tub 2.

A heating device 14 is also provided in the outer tub 2. The valve 10 and the heating device 14 can be controlled by a program control system 13 depending on a program execution plan which can be linked to a time program and/or to the reaching of particular measured values of parameters such as the suds level, the suds temperature, the rotary speed of the drum 3, etc. within the washing machine 1. 16 denotes a pump for the liquid, in particular washing suds 7, in the outer tub 2.

A water reservoir 18 can store the greywater which has been used for rinsing the laundry item 8. Said greywater can be used for a later washing cycle. For this purpose, the water reservoir 18 is connected via a line ("feed pipe for rinsing liquid") 19 to the valve 10 which also regulates the fresh water feed. A circulating element 20 and an air infeed element 21, which can also be controlled by the program control system 13 are provided in the water reservoir 18. With these measures, a particularly efficient oxidation reaction is possible at the interior surface 17 in the water reservoir 18.

Interior surfaces 17 which contain A₃PW₄O₂₄, A₅PV₂Mo₁₀O₄₀ or A₄PVW₁₁O₄₀, wherein A is either the cation methyltrioctylammonium or tributyltetradecylphosphoinium are applied in the detergent dispenser tray 12, in the outer tub 2 and in the water reservoir 18.

### REFERENCE NUMERALS

- 1: Household appliance, water-bearing household appliance, washing machine
- 2: (Outer) tub
- 3: Laundry drum
- 4: Rotation axis of the drum
- 5: Laundry agitators
- 6: Scooping devices
- 7: Washing suds
- 8: Laundry items
- 9: Domestic water supply
- 10: Valve
- 11: Feed pipe
- 12: Detergent dispenser tray
- 13: Program control system
- 14: Heating device
- 15: Drive motor
- 16: Pump
- 17: Interior surface containing polyoxometalate
- 18: Water reservoir
- 19: Feed pipe for rinsing water
- 20: Circulating element
- 21: Air infeed element

## Claims

1. A household appliance (1) with at least one polyoxometalate containing surface (17), **characterized in that** the catalytically active substance is a polyoxometalate of the Formula (I) or (II),
A_{q+3}PV_{q}Z_{12-q}O₄₀ (I),
A₃PZ₄O₂₄ (II)
wherein Z is selected from Mo oder W,
q is 0, 1, 2 or 3, and
A is selected from one or more cations selected from the group consisting of (XR¹R²R³R⁴)⁺, wherein at least one of the groups R¹, R², R³, and R⁴ is a substituted or nonsubstituted alkyl or cycloalkyl group with at least five carbon atoms and wherein X is selected from nitrogen or phosphorus.

2. The household appliance (1) of claim 1, wherein the polyoxometalate is a polyoxometalate of the Formula (I) or (II),
A_{q+3}PVqMo_{12-q}O₄₀ (I'),
A₃PW₄O₂₄ (II'),
or
A_{q+3}PV_{q}W_{12-q}O₄₀ (III'),
wherein q is 0, 1 or 2.

3. The household appliance (1) of claim 2, wherein the polyoxometalate is
A_{q+3}PV_{q}Mo_{12-q}O₄₀ (I'),
or
A_{q+3}PV_{q}W_{12-q}O₄₀ (III'),
wherein q is 1 or 2.

4. The household appliance (1) of claim 2, wherein the polyoxometalate is
A₃PW₄O₂₄ (II')

5. The household appliance (1) of any of claims 1 to 4, wherein the household appliance is a water-bearing household appliance (1) and the surface (17) is an interior surface (17) provided in the interior of the household appliance (1) which comes into contact with flowing or static water (7) during operation of the household appliance (1).

6. The household appliance (1) of claim 5, wherein the at least one interior surface (17) is provided in the water feed system (9,10,11).

7. The household appliance (1) of claims 5 or 6, wherein the at least one interior surface is provided in a water reservoir (18).

8. The household appliance (1) of claim 7, wherein the water reservoir (18) comprises a circulating element (20) and/or an air infeed element (21).

9. The household appliance (1) of any of claims 1 to 8, wherein the household appliance is a dishwater or a laundry treatment device or a coffee making machine.

10. The household appliance (1) of claim 9, wherein the household appliance is a laundry treatment device belonging to the group consisting of a washing machine (1) and a washer-dryer.

11. The household appliance (1) of any of claims 1 to 10, wherein the cation is selected from one or more cations selected from the group consisting of (NR¹R²R³R⁴)⁺, wherein at least one of the groups R¹, R², R³, and R⁴ is a substituted or nonsubstituted alkyl or cycloalkyl group with at least eight carbon atoms.

12. The household appliance (1) of any of claims 1 to 10, wherein the cation is selected from one or more cations selected from the group consisting of (PR¹R²R³R⁴)⁺, wherein at least one of the groups R¹, R², R³, and R⁴ is a substituted or nonsubstituted alkyl or cycloalkyl group with at least eight carbon atoms.

13. The household appliance (1) of any of claims 1 to 12, wherein the surface (17) has a polyoxometalate containing upper layer with a thickness of from 0.01 to 1.5 mm.

14. The household appliance (1) of claim 13, wherein the upper layer of the surface (17) has a polyoxometalate content in the range of from 0.5 to 50 wt%, based on the weight of the upper layer.

15. A method for operating a household appliance (1), wherein the household appliance is a water-bearing household appliance comprising a tub (2) for accommodating articles (8) to be cleaned and at least one catalytically active polyoxometalate-containing interior surface (17), which can come into contact with water or washing suds (7) during operation of the household appliance, and wherein the polyoxometalate is of the Formula (I) or (II),
A_{q+3}PV_{q}Z_{12-q}O₄₀ (I),
A₃PZ₄O₂₄ (II)
wherein Z is selected from Mo oder W,
q is 0, 1, 2 or 3, and
A is selected from one or more cations selected from the group consisting of (XR¹R²R³R⁴)⁺, wherein at least one of the groups R¹, R², R³, and R⁴ is a substituted or nonsubstituted alkyl or cycloalkyl group with at least five carbon atoms and wherein X is selected from nitrogen or phosphorus,
and wherein water or washing suds (7) to be cleaned is brought into contact with the polyoxometalate-containing surface (17).
